(19) Europäisches Patentamt
European Patent Office
Office européen des brevets

(11) EP 2 799 115 A1

# (12) EUROPEAN PATENT APPLICATION

(43) Date of publication:
**05.11.2014 Bulletin 2014/45**

(21) Application number: **13166427.8**

(22) Date of filing: **03.05.2013**

(51) Int Cl.:
***A61Q 5/10*** (2006.01)
***A61K 8/19*** (2006.01)
***A61K 8/365*** (2006.01)
***A61K 8/368*** (2006.01)
***A61K 8/37*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Unilever PLC**
**London, Greater London EC4Y 0DY (GB)**

(72) Inventors:
- **Ainger, Nocholas John**
**Bebington, Wirral Merseyside CH63JW (GB)**
- **Batchelor, Stephen Norman**
**Bebington, Wirral Merseyside CH63JW (GB)**
- **Fournier, Charlene**
**Bebington, Wirral Merseyside CH63JW (GB)**

(74) Representative: **James, Helen Sarah et al**
**Unilever Patent Group**
**Colworth House**
**Sharnbrook**
**Bedford MK44 1LQ (GB)**

## (54) Hair care composition

(57) A kit for colouring hair comprising:
i) a first composition comprising a polyphenol
ii) a second composition comprising a transition metal salt and/or complex, such that the oxygen concentration in the second composition is less than 2ppm.

EP 2 799 115 A1

## Description

The present invention relates to colouring systems; in particular colouring systems based on a polyphenol/metal salt system.

## Background of the Invention

Two part colouring systems involving a metal ion and a polyphenol are described in EP0327345 (Beecham) and WO2010/135237 (Advance Cosmetic Technologies).

JP 04/164,017 (Lion) discloses a system for colouring hair consisting of shampoo containing a surfactant and gallic acid, tannic acid, salicylic acid, derivatives thereof, pyrogallol, catechol or haematin as a first agent. This is used in conjunction with a second agent containing a polyvalent metal salt and a cationic surfactant.

The present system provides more effective colouring of human hair from a two part metal and polyphenol system.

## Description of the Invention

Accordingly the present invention provides a kit for colouring hair comprising:

i) a first composition comprising a polyphenol
ii) a second composition comprising a transition metal salt and/or complex, such that the oxygen concentration in the composition is less than 2ppm.

The invention further relates to a method of colouring hair comprising the steps of sequentially applying the mentioned composition.

## Detailed Description of the Invention

### First Composition

Polyphenol

The first composition of the kit comprises a polyphenol. The polyphenol of the invention comprises at least one benzene ring substituted with a first OH group and a second OH or $OCH_3$ group, preferably OH. Preferably the first OH is ortho to the second OH group.

The OH containing ring may be fused to aromatic or non-aromatic rings. Polyphenols may be found in the classes of phenolic acid, anthocyanin, anthocyanidin, flavanols, flavanones and gallates. For example flavan-3-ols, flavan-4-ols, flavan-3,4-ols.

It is highly preferable if the polyphenol is not an anthraquinone, thus the polyphenol is not derived or based on henna.

A preferred polyphenol contains a benzene ring substituted in the:

1 position by an X group, wherein X is a carbon containing organic group, preferably selected from an ester group, COOH, chromenes and 3,4 dihydro-chromenes. 3 position by a H, OH or $OCH_3$ group, preferably an OH group. 4 position by an OH group. 5 position by an OH or $OCH_3$ group, preferably an OH group.

Most preferably the 2 and 6 positions are substituted by H.

Preferably the polyphenol is an ortho-dihydroxybenzene derivative.

Preferably the polyphenol has a molecular weight of less than 1,000, more preferably less than 500.

The polyphenol of the invention is preferably selected from gallic acid, methyl gallate, ethyl gallate, propyl gallate or mixtures thereof, most preferably propyl gallate

Methyl gallate, ethyl gallate, propyl gallate are obtainable by esterification of gallic acid. Preferably the gallic acid is extracted from a natural source, preferably from hydrolysable tannins, preferably extracted from tara plants or gallnuts. Preferably, the polyphenols do not contain any active oxidase enzymes and have been heat treated to destroy any enzyme activity.

The polyphenol containing composition is preferably an aqueous polyphenol solution. The polyphenol composition preferably comprises from 0.01 wt% to 10.0 wt% of the total first composition, more preferably from 0.2 wt% to 4.0 wt %, most preferably 0.5 wt% to 2.5 wt% of polyphenol.

To avoid oxidation of the polyphenols by air it is preferred that the composition is stored in an airtight container, preferably a bottle closed with an air tight cap.

The aqueous polyphenol solution contains water, preferably as the dominant ingredient. Auxiliary ingredients may be present for example to increase the viscosity, perfume and help solubilise the polyphenol. Solubilising ingredients include organic solvents and surfactants.

Preferably, the water used to formulate all compositions has a French hardness of from 0 to 36 degrees, more preferably 0 to 24 degrees, most preferably from 0 to 2 degrees.

Preferably, the water used to formulate all compositions contains less than 1ppm of chlorine based bleaching agents such as chlorine dioxide or hypochlorite. Most preferably less than 50ppb.

**<u>Second Composition</u>**

<u>Metal</u>

The second composition of the invention at least one metal ion of in which the metal is selected from iron, copper, zinc or manganese. The metal ion is preferably present in the form of a salt or a complex.

Preferably, the metal ion is Fe(II), Fe(III) or Cu(II), more preferably the metal ion is selected from Fe(II), Fe(III), most preferably Fe(II).

Preferably the level of metal ion in the total second composition is from 0.01wt% to 5 wt% of metal ion, more preferably 0.05 to 4 wt% more preferably from 0.4 to 1 wt%.

Preferably the fraction of [Fe(III)]/([Fe(II)]+[Fe(III)]) is less than 0.05, more preferably less than 0.01, most preferably less than 0.001. [Fe] is the concentration of the iron ion in mol/L.

For the avoidance of doubt, if the formulation contains 2wt% of the complex

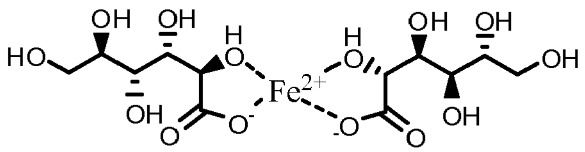

With molecular weight of 446.14, then it will contain 55.84/446.14*2 = 0.25wt% of Fe(II) (2 decimal places).

The iron ion is preferably present as a complex with a ligand. The ligand is preferably gluconate and/or lactate. The ligand-metal complex may be pre-formed before addition to the shampoo for example iron (II) gluconate. The ligand metal complex may be formed in the composition by the addition of an iron salt and the ligand for example, $FeCl_2$ and sodium lactate or tartrate. Preferably, the mole ratio of iron ion to ligand is from 1:1 to 1:4, more preferably from 1:1.5 to 1:2.5.

Preferably, the metal, more preferably iron ion is in the form of a complex that is soluble in aqueous solution at the pH of the formulation. Most preferably the iron ion in the form of a salt and/or complex has a solubility in demineralised water at the pH of the formulation of greater than 0.1 g/L.

The ability of a ligand to complex with a metal ion, $M^{n+}$, may be defined by its $pM^{n+}$ value, wherein

$$pM^{n+} = -log_{10}[M^{n+}]_{free}$$

and the ligand concentration is $10^{-5}$ mol/L and the total metal ion concentration is $10^{-6}$ mol/L and $[M^{n+}]_{free}$ is the molar concentration of uncomplexed metal ions. Preferably the ligand used has a lower $pM^{n+}$, than gallic acid. Preferably the $pM^{n+}$ are greater than 8 more preferably greater than 10. $pM^{n+}$ values are most preferably measured at pH 4 in demineralised water, with the chloride salt of the metal ion, most preferably ferric chloride.

Polyamino carboxylic acids *are preferably present at weight % levels of less than 1/30th of the main ligand, preferably they are absent from the iron containing formulation.* A polyamino carboxylic acid is a compound containing two or more <u>amines</u> connected through <u>carbon</u> atoms to two or more <u>carboxyl</u>ic acid groups. 2,2',2'',2'''-(Ethane-1,2-diyldinitrilo)tetraacetic acid is a polyamino carboxylic acid. Diethylene triamine pentaacetic acid is a polyamino carboxylic acid. Ethylenediamine-N,N'-disuccinic acid is a polyamino carboxylic acid.

Particularly preferred are iron (II) gluconate and iron(II) lactate.

The composition comprising the meta ion has an oxygen concentration in the composition of less than 2ppm,

preferably less than 1ppm, more preferably less than 0.1ppm. Preferably this is achieved by nitrogen purging. Preferably this is done before the metal salt/complex (iron(II) salt and/or complex) is dissolved in the formulation. If nitrogen purging is completed after dissolution of the metal salt it is preferable if this occurs immediately after the iron(II) salt and/or complex is added.

Preferably, the metal is in a composition which further comprises a cationic surfactant, such as a conditioner composition.

Product Form

Compositions of the invention are typically "rinse-off" compositions to be applied to the hair and then rinsed away.

Shampoo Composition

Shampoo compositions of the invention are generally aqueous, i.e. they have water or an aqueous solution or a lyotropic liquid crystalline phase as their major component. Suitably, the composition will comprise from 50 % to 98%, preferably from 60 % to 90% water by weight based on the total weight of the composition.

**Anionic Cleansing Surfactant**

Shampoo compositions according to the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate(n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 % to 45%, preferably from 1.5 % to 35%, more preferably from 5 % to 20 % by total weight anionic cleansing surfactant based on the total weight of the composition.

**Further Ingredients**

Optionally, a shampoo composition of the invention may contain further ingredients as described below to enhance performance and/or consumer acceptability.

**Co-surfactant**

The composition can include co-surfactants, to help impart aesthetic, physical or cleansing properties to the composition.

An example of a co-surfactant is a nonionic surfactant, which can be included in an amount ranging from 0.5 % to 8%, preferably from 2 % to 5% by weight based on the total weight of the composition.

For example, representative nonionic surfactants that can be included in shampoo compositions of the invention include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco monoisopropanolamide.

Further nonionic surfactants which can be included in shampoo compositions of the invention are the alkyl polyglycosides (APGs). Typically, APG is one which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group.

R may represent a mean alkyl chain length of from about $C_5$ to about $C_{20}$. Preferably R represents a mean alkyl chain length of from about $C_8$ to about $C_{12}$. Most preferably the value of R lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the $C_{10}$-$C_{18}$ N-alkyl ($C_1$-$C_6$) polyhydroxy fatty acid amides, such as the $C_{12}$-$C_{18}$ N-methyl glucamides, as described for example in WO 92 06154 and US 5 194 639, and the N-alkoxy polyhydroxy fatty acid amides, such as $C_{10}$-$C_{18}$ N-(3-methoxypropyl) glucamide.

A preferred example of a co-surfactant is an amphoteric or zwitterionic surfactant, which can be included in an amount ranging from 0.5% to about 8%, preferably from 1 % to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocoamidopropyl betaine.

Mixtures of any of the foregoing amphoteric or zwitterionic surfactants may also be suitable. Preferred mixtures are those of cocoamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1% to 50%, preferably from 2 % to 40%, more preferably from 10 % to 25% by total weight surfactant based on the total weight of the composition.

## Cationic Polymers

Cationic polymers are preferred ingredients in a shampoo composition of the invention for enhancing conditioning performance.

Suitable cationic polymers may be homopolymers which are cationically substituted or may be formed from two or more types of monomers. The weight average ($M_w$) molecular weight of the polymers will generally be between 100,000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;

- mineral acid salts of amino-alkyl esters of homo-and co-polymers of unsaturated carboxylic acids having from 3 to 5 carbon atoms, (as described in U.S. Patent 4,009,256);

- cationic polyacrylamides (as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

$$A\text{-}O\text{-}[R\text{-}N+(R1)(R^2)(R^3)X^-],$$

wherein: A is an anhydroglucose residual group, such as a starch or cellulose anhydroglucose residual. R is an alkylene, oxyalkylene, polyoxyalkylene, or hydroxyalkylene group, or combination thereof. $R^1$, $R^2$ and $R^3$ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in $R^1$, $R^2$ and $R^3$) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 % to 5%, preferably from 0.05 % to 1 %, more preferably from 0.08 % to 0.5% by total weight of cationic polymer based on the total weight of the composition.

**Suspending Agent**

Preferably, an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Lubrizol.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer

of acrylic acid and crystalline long chain acyl derivative.

Suspending agent will generally be present in a shampoo composition of the invention at levels of from 0.1 % to 10%, preferably from 0.5 % to 6%, more preferably from 0.9 % to 4% by total weight of suspending agent based on the total weight of the composition.

Conditioner Compositions

One part of the kit may be a conditioner for the treatment of hair (typically after shampooing) and subsequent rinsing. Such conditioner compositions preferably comprise the polyphenol.

Such conditioner compositions will typically comprise one or more conditioning surfactants which are cosmetically acceptable and suitable for topical application to the hair.

Suitable conditioning surfactants include those selected from cationic surfactants, used singly or in admixture. Preferably, the cationic surfactants have the formula $N{+}R^1R^2R^3R^4$ wherein $R^1$, $R^2$, $R^3$ and $R^4$ are independently ($C_1$ to $C_{30}$) alkyl or benzyl. Preferably, one, two or three of $R^1$, $R^2$, $R^3$ and $R^4$ are independently ($C_4$ to $C_{30}$) alkyl and the other $R^1$, $R^2$, $R^3$ and $R^4$ group or groups are ($C_1$-$C_6$) alkyl or benzyl. More preferably, one or two of $R^1$, $R^2$, $R^3$ and $R^4$ are independently ($C_6$ to $C_{30}$) alkyl and the other $R^1$, $R^2$, $R^3$ and $R^4$ groups are ($C_1$-$C_6$) alkyl or benzyl groups. Optionally, the alkyl groups may comprise one or more ester (-OCO- or - COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:

(i) an amidoamine corresponding to the general formula (I):

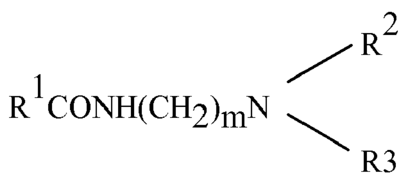

in which $R^1$ is a hydrocarbyl chain having 10 or more carbon atoms,
$R^2$ and $R^3$ are independently selected from hydrocarbyl chains of from 1 to 10 carbon atoms, and
m is an integer from 1 to about 10; and

(ii) an acid.

As used herein, the term hydrocarbyl chain means an alkyl or alkenyl chain.

Preferred amidoamine compounds are those corresponding to formula (I) in which
$R^1$ is a hydrocarbyl residue having from about 11 to about 24 carbon atoms,
$R^2$ and $R^3$ are each independently hydrocarbyl residues, preferably alkyl groups, having from 1 to about 4 carbon atoms, and m is an integer from 1 to about 4.

Preferably, $R^2$ and $R^3$ are methyl or ethyl groups.

Preferably, m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

A protonating acid may be present. Acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof.

Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate more than 95 mole% (293 K) of the amidoamine present.

In conditioners of the invention, the level of cationic surfactant will generally range from 0.01 % to 10%, more preferably 0.05 % to 7.5%, most preferably 0.1 % to 5% by weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed. Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 % to 10%, preferably from 0.1 % to 8%, more preferably from 0.2 % to 7%, most preferably from 0.3% to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

## Further Conditioning Agents

Compositions of the invention may comprise further conditioning agents to optimise wet and dry conditioning benefits.

The compositions of the invention can contain, emulsified droplets of a silicone conditioning agent, for enhancing conditioning performance. Silicone conditioning agents may be present in the shampoo or conditioner.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25°C the viscosity of the silicone itself is preferably at least 60,000 cst, most preferably at least 500,000 cst, ideally at least 1,000,000 cst. Preferably the viscosity does not exceed $10^9$ cst for ease of formulation.

Emulsified silicones for use in the shampoo compositions of the invention will typically have an average silicone droplet size in the composition of less than 30, preferably less than 20, more preferably less than 10 $\mu$m, ideally from 0.01 to 1 $\mu$m. Silicone emulsions having an average silicone droplet size of $\leq 0.15$ $\mu$m are generally termed microemulsions.

Emulsified silicones for use in the conditioner compositions of the invention will typically have a size in the

composition of less than 30, preferably less than 20, more preferably less than 15. Preferably, the average silicone droplet is greater than 0.5 μm, more preferably greater than 1 μm, ideally from 2 to 8 μm.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone", Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

With some shampoos it is preferred to use a combination of amino and non amino functional silicones.

The total amount of silicone is preferably from 0.01 wt% to 10 %wt of the total composition more preferably from 0.1 wt% to 5 wt%, most preferably 0.5 wt% to 3 wt% is a suitable level, especially for a shampoo composition.

<u>Other Optional Ingredients</u>

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

<u>Method of use</u>

The method of colouring hair comprising the steps of applying to hair sequentially in any order:

(i) a shampoo composition comprising at least one metal salt and/or complex in which the metal is selected from iron, copper, zinc or manganese and a surfactant mix comprising from 1.5 wt% to 50 wt% of the total shampoo composition of a nonionic in which the nonionic surfactant is the major component by weight of the surfactant mix and

(ii) a second composition, which comprises from 0.05 wt% to 10 wt% of the total second composition of a polyphenol.

Preferably, the compositions of the invention are applied to wet hair, necessitating the step of wetting the hair before application of the compositions of the invention.

A method preferably comprises the step of rinsing hair between application of the shampoo composition (i)and the second composition (ii). It is highly preferred if the hair is rinsed after application of both compositions of the invention.

Further conditioning and/or styling products may be applied as part of the colouring process.

The level of each composition applied to the head of hair is preferably from 5g to 100g.

Preferably, each composition remains on the hair for 5 to 600 seconds, more preferably 10 to 300 seconds.

Preferably, the water used to wet and rinse the hair has a French hardness of from 0 to 36 degrees, more preferably 0 to 24 degrees, most preferably from 0 to 2 degrees.

Preferably, the water used to wet and rinse the hair contains less than 1 ppm of chlorine based bleaching agents such as chlorine dioxide or hypochlorite. Most preferably less than 50ppb

The invention will now be illustrated by the following non-limiting examples.

**<u>Examples</u>**

**<u>Example 1</u>**

4 wt% of iron (II) gluconate was dissolved in water and the solution immediately bubbled with a continuous flow of nitrogen gas, ensuring complete mixing of the solution for 1 hour. 3 Chinese human white hair switches were added

to the solution for a further 1 hour with continuous nitrogen flow. The hair switches were removed, rinsed (30 seconds) and washed with 3g of the shampoo formulation for 2 mins and then rinsed (30 seconds) and dried in air. The colour of the switches were measured with a reflectometer and expressed as the CIE L*a*b* values. The experiment was repeated, except the nitrogen was replaced with compressed air (control).

**Shampoo**

| **Ingredient** | wt% |
|---|---|
| Sodium Laureth Sulfate | 17.0 |
| Cocamidopropylbetaine | 5.3 |
| Carbomer | 0.4 |
| Hydrochloric acid | 0.06 |
| Mica | 0.15 |
| DMDM Hydantoin | 0.1 |
| Silicone | 1.7 |
| Guar Hydroxypropyltrimonium Chloride | 0.2 |
| Propyl gallate | 2 |
| Sodium Chloride | 0.5 |
| NaOH | To pH = 6.5 |
| water | to 100 |

The results are summarised in the table below

| | L* | a* | b* |
|---|---|---|---|
| Before treatment | 71.0 | 3.1 | 25.2 |
| Nitrogen | 38.8 | 1.8 | 1.2 |
| Air (control) | 48.4 | 1.3 | 5.5 |

The nitrogen purged samples provide a much greater darker colour than the control, as shown by the lower L* values.

## Claims

1. A kit for colouring hair comprising:

   i) a first composition comprising a polyphenol
   ii) a second composition comprising a transition metal salt and/or complex, such that the oxygen concentration in the second composition is less than 2ppm.

2. A kit according to any preceding claim in which the transition metal of the second composition ii) is selected from iron or copper.

3. A kit according to claim 3 in which the transition metal of the second composition ii) is iron.

4. A kit according to claim 3 in which the iron is present as a complex of gluconate and/or lactate.

5. A kit according to any preceding claim in which iron is present as iron (II) gluconate.

6. A kit according to any preceding claim in which composition i) comprising the polyphenol is a shampoo composition and further comprises anionic surfactant.

7. A kit according to any preceding claim in which the composition ii) comprising the metal ion is a conditioner composition and further comprises a cationic surfactant.

8. A kit according to any preceding claim in which the pH of the first composition is from 3 to 8 at 20°C.

9. A kit according to in any preceding claim in which the polyphenol is selected selected from methyl gallate, ethyl gallate, propyl gallate and mixtures thereof.

10. A kit according to preceding claim in which the level of polyphenol is from 0.2 wt% to 4.0 wt % of the total first composition.

11. A kit according to any preceding claim in which the level of metal ion is from 0.01wt% to 5 wt% of the total second composition.

12. A method of colouring hair comprising the steps of sequentially applying the compositions of the kit described in the above claims to the hair.

Europäisches Patentamt
European Patent Office
Office européen des brevets

# EUROPEAN SEARCH REPORT

Application Number
EP 13 16 6427

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 0 327 345 A2 (BEECHAM GROUP PLC [GB]) 9 August 1989 (1989-08-09) * claims 1,2,4,9 * * paragraphs [0004], [0011] * | 1-12 | INV. A61Q5/10 A61K8/19 A61K8/365 A61K8/368 A61K8/37 |
| X | WO 2012/139948 A2 (UNILEVER PLC [GB]; UNILEVER NV [NL]; UNILEVER HINDUSTAN [IN]; AINGER N) 18 October 2012 (2012-10-18) * page 1, line 21 - page 4, line 2 * * page 4, line 9 - line 10 * | 1-12 | |
| X,D | JP H04 164017 A (LION CORP) 9 June 1992 (1992-06-09) * abstract * | 1-12 | |

TECHNICAL FIELDS SEARCHED (IPC)
A61Q
A61K

The present search report has been drawn up for all claims

2

| Place of search | Date of completion of the search | Examiner |
|---|---|---|
| Munich | 24 October 2013 | Simon, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 6427

This annex lists the patent family members relating to the patent documents cited in the above-mentioned European search report. The members are as contained in the European Patent Office EDP file on
The European Patent Office is in no way liable for these particulars which are merely given for the purpose of information.

24-10-2013

| Patent document cited in search report | Publication date | Patent family member(s) | Publication date |
|---|---|---|---|
| EP 0327345 A2 | 09-08-1989 | AU 2954289 A | 10-08-1989 |
| | | DE 68909963 D1 | 25-11-1993 |
| | | DE 68909963 T2 | 24-02-1994 |
| | | DK 48789 A | 05-08-1989 |
| | | EP 0327345 A2 | 09-08-1989 |
| | | ES 2060748 T3 | 01-12-1994 |
| | | JP H01308218 A | 12-12-1989 |
| | | MA 21488 A1 | 01-10-1989 |
| | | NZ 227843 A | 21-12-1989 |
| | | PH 25284 A | 30-04-1991 |
| | | PT 89596 A | 04-10-1989 |
| | | ZA 8900821 A | 27-12-1989 |
| | | ZM 889 A1 | 30-06-1989 |
| | | ZW 1389 A1 | 18-10-1989 |
| WO 2012139948 A2 | 18-10-2012 | NONE | |
| JP H04164017 A | 09-06-1992 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0327345 A, Beecham
- WO 2010135237 A
- JP 4164017 A, Lion
- WO 9206154 A
- US 5194639 A
- US 4009256 A
- WO 9522311 A
- US 3962418 A
- US 3958581 A
- WO 9631188 A
- EP 0530974 A